# EUROPEAN PATENT SPECIFICATION

(11) **EP 3 771 498 B1**
(45) Date of publication and mention of the grant of the patent: **16.07.2025**
(21) Application number: 20188895.5
(22) Date of filing: 31.07.2020
(51) Int. Cl.: B06B 1/06

(54) **ULTRASOUND PROBE AND ULTRASOUND DIAGNOSIS APPARATUS**
ULTRASCHALLSONDE UND ULTRASCHALLDIAGNOSEVORRICHTUNG
SONDE À ULTRASONS ET APPAREIL DE DIAGNOSTIC À ULTRASONS

(30) Priority: 31.07.2019 JP 2019141063
(43) Date of publication of application: 03.02.2021
(73) Proprietor: Canon Medical Systems Corporation, Otawara-shi, Tochigi 324-0036 (JP)
(72) Inventor: SHIKATA, Hiroyuki, Tochigi, 324-0036 (JP)
(74) Representative: Marks & Clerk LLP

(56) References cited:
- JP-A- S57 132 497
- JP-A- S60 114 239
- US-A- 4 641 660
- US-A1- 2009 270 735
- US-A1- 2016 007 964
- US-B2- 9 629 608

## Description

### BACKGROUND

Ultrasound examinations are carried out by a medical technologist or a medical doctor operating an ultrasound probe on the body surface of an examined subject (hereinafter "patient") so as to obtain information about a tissue structure, blood flows, and the like inside the human body. For example, the medical technologist or the medical doctor scans the inside of the patient's body with ultrasound waves by operating, on the body surface, the ultrasound probe configured to transmit and receive ultrasound waves in accordance with the diagnosed site and/or specifics of a diagnosis, to acquire ultrasound images indicating the tissue structure or ultrasound images indicating the information about the blood flows or the like.

As ultrasound probes used for these ultrasound examinations, for example, two-dimensional array probes are known. A two-dimensional array probe includes a transducer element array in which plate-like transducer elements are divided in sections in a grid formation (hereinafter, "divided in grid sections"). Because the two-dimensional array probe has a large number of elements (the number of transducer elements after the division), a driving circuit is provided in the vicinity of the transducer elements so that, at the time of reception, signals in a limited quantity are connected to an apparatus main body by performing a partial beam forming process.

Further, another technique is also known by which a transducer element array divided in grid sections is connected to an apparatus as an annular array, by being provided with electrodes in a formation of multiple concentric circles. Because the annular array is unable to scan a cross-section by deflecting or moving an ultrasound beam, it is necessary to perform a scan by mechanically swinging the transducer elements. However, the beam form is excellent and the obtained images have high quality.

Japanese patent application JPS57132497A relates to a disc shape ceramic piezoelectric plate on one surface of which a common electrode is bonded which is diced longitudinally and transversally. A non-conductive sound wave absorbing member is filled in cut-groove formed with the dicing and circular-arc shaped electrodes are formed on the surface by coating or printing conductive material.

Japanese patent application JPS60114239A relates to an ultrasonic probe using a composite piezoelectric vibrator suitable for a vibrator material.

United States patent application US 2009/270735 A1 relates to an electronic array probe for ultrasonic imaging including an array of transmitting and/or receiving electroacoustic transducers arranged in concentric bands in which the transducers are tangent to one other in both radial and circumferential directions.

United States patent application US4641660A relates to an echography apparatus comprising a probe reconstituting mobile rings by element switching, said probe comprising a plurality of transducer elements spread over a convex coupling surface, and switching means being provided for grouping together certain transducer elements into rings.

United States Patent Application US2016/0007964 A1 discloses an ultrasonic probe which comprises an ultrasonic transducer array formed by arraying a plurality of ultrasonic transducers provided with electrodes on end faces and a multilayer body configured to extract electric wirings from the respective electrodes.

### SUMMARY OF INVENTION

In a first aspect, an ultrasound probe is provided as recited in claim 1.

In addition to a first FPC serving as the substrate, a second FPC may further be provided so as to be provided on a rear surface of the first FPC, to have a conductive part in a position corresponding to the position of a conductive part of the first FPC, and to be configured to transmit and receive a signal to and from the first FPC. Between the first FPC and the second FPC, the conductive parts may be made conductive with each other via a conductive joint, while gaps other than conductively joined positions are filled with a non-conductive adhesive agent.

Each of groups of piezoelectric transducer elements corresponding to electrodes located in substantially concentric circles may be connected in parallel to one or more driving circuits.

Among the groups of piezoelectric transducer elements, a ratio between the quantity of piezoelectric transducer elements and the quantity of driving circuits may be kept substantially constant.

For groups of piezoelectric transducer elements each connected to two or more driving circuits, average distances from the center of the plurality of piezoelectric transducer elements may be kept at substantially constant intervals.

A swinging unit configured to swing the plurality of piezoelectric transducer elements divided in the grid sections and the substrate may further be provided.

In a second aspect, an ultrasound diagnosis apparatus is provided as recited in claim 7.

A swinging mechanism configured to swing the ultrasound probe may further be provided.

### BRIEF DESCRIPTION OF THE DRAWINGS

FIG. 1 is block diagram illustrating an exemplary configuration of an ultrasound diagnosis apparatus according to a first embodiment;
FIG. 2 is a drawing illustrating an exemplary configuration of an ultrasound probe according to the first embodiment;
FIG. 3A is a drawing for explaining a first Flexible Preprint Circuit (FPC) according to the first embodiment;
FIG. 3B is another drawing for explaining the first FPC according to the first embodiment;
FIG. 4 is a drawing for explaining channels of the ultrasound probe according to the first embodiment;
FIG. 5 is a drawing for explaining connections to transmission and reception circuits from the ultrasound probe according to the first embodiment;
FIG. 6 is a chart illustrating a relationship between the quantity of transducer elements and average distances in the ultrasound probe according to the first embodiment;
FIG. 7 is a drawing illustrating an example of a swinging mechanism according to the first embodiment;
FIG. 8 is a flowchart for explaining a procedure in a process performed by the ultrasound diagnosis apparatus according to the first embodiment; and
FIG. 9 is a drawing illustrating an exemplary configuration of an ultrasound probe according to a second example, which does not form part of the claimed invention.

### DETAILED DESCRIPTION

According to an embodiment, an ultrasound probe includes a plurality of piezoelectric transducer elements and a substrate. The plurality of piezoelectric transducer elements is divided in grid sections. The substrate is laid on a rear surface of the plurality of piezoelectric transducer elements and has a wiring configured to cause a plurality of electrodes for the plurality of piezoelectric transducer elements to be connected in parallel to driving circuitry in a formation of substantially concentric circles, the rear surface of the plurality of piezoelectric transducer elements being a surface opposite to ultrasound wave transmitting/receiving surface.

Exemplary embodiments of an ultrasound probe and an ultrasound diagnosis apparatus of the present disclosure will be explained in detail below, with reference to the accompanying drawings. The ultrasound probe and the ultrasound diagnosis apparatus of the present disclosure are not limited to the embodiments described below. Further, in the following explanations, some of the constituent elements that are the same as one another will be referred to by using the same reference characters, and the duplicate explanations thereof will be omitted.

### First Embodiment

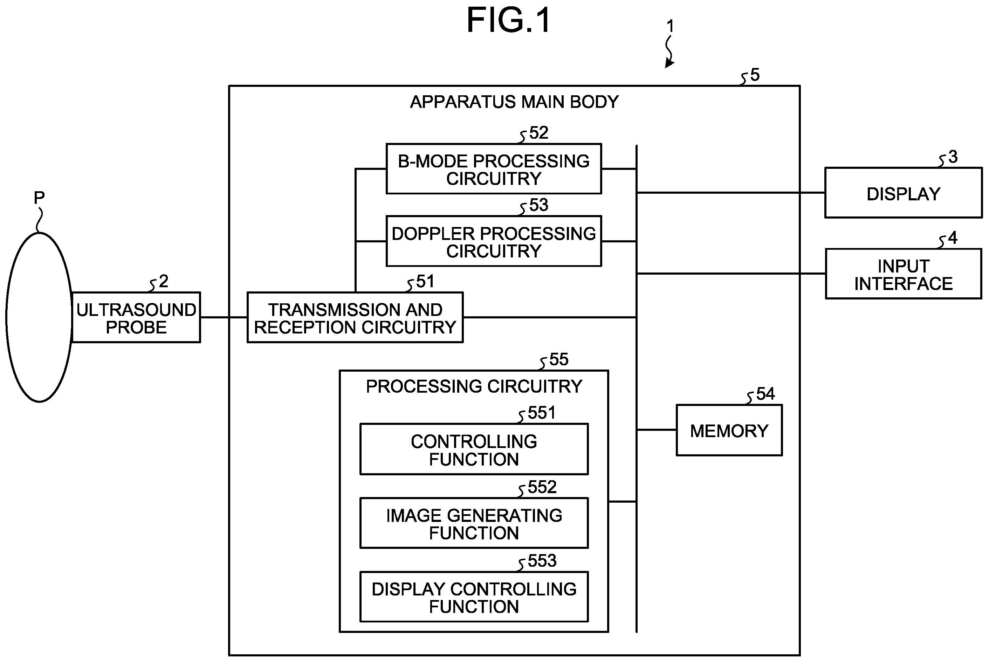

To begin with, an ultrasound diagnosis apparatus according to a first embodiment will be explained. FIG. 1 is a block diagram illustrating an exemplary configuration of an ultrasound diagnosis apparatus 1 according to the first embodiment. As illustrated in FIG. 1, the ultrasound diagnosis apparatus 1 according to the present embodiment includes an ultrasound probe 2, a display 3, an input interface 4, and an apparatus main body 5. The ultrasound probe 2, the display 3, and the input interface 4 are communicably connected to the apparatus main body 5.

The ultrasound probe 2 is connected to transmission and reception circuitry 51 included in the apparatus main body 5. For example, in the probe main body, the ultrasound probe 2 includes a plurality of piezoelectric transducer elements. The plurality of piezoelectric transducer elements are configured to generate ultrasound waves on the basis of drive signals supplied thereto from the transmission and reception circuitry 51. Further, the ultrasound probe 2 is configured to receive reflected waves from a subject P and to convert the received reflected waves into electrical signals. Further, in the probe main body, the ultrasound probe 2 includes a matching layer provided for the piezoelectric transducer elements, as well as a backing member or the like that prevents the ultrasound waves from propagating rearward from the piezoelectric transducer elements. The ultrasound probe 2 is detachably connected to the apparatus main body 5. In this situation, the ultrasound probe 2 according to the present embodiment is an ultrasound probe in which a transducer element array divided in grid sections is connected in a formation of multiple concentric circles with excellent area efficiency. This aspect will be explained in detail later.

When an ultrasound wave is transmitted from the ultrasound probe 2 to the subject P, the transmitted ultrasound wave is repeatedly reflected on a surface of discontinuity of acoustic impedances at a tissue in the body of the subject P and is received as a reflected-wave signal by the plurality of piezoelectric transducer elements included in the ultrasound probe 2. The amplitude of the reflected-wave signal is dependent on the difference between the acoustic impedances on the surface of discontinuity on which the ultrasound wave is reflected. When a transmitted ultrasound pulse is reflected on the surface of a moving blood flow, a cardiac wall, or the like, the reflected-wave signal is, due to the Doppler effect, subject to a frequency shift, depending on a velocity component of the moving members with respect to the ultrasound wave transmission direction.

The display 3 is configured to display a Graphical User Interface (GUI) used by an operator of the ultrasound diagnosis apparatus 1 for receiving inputs of various types of setting requests via the input interface 4 and to display ultrasound images, display information, and the like generated in the apparatus main body 5. Further, to inform the operator of processing statuses and processing results of the apparatus main body 5, the display 3 is configured to display various types of messages and display information. The display 3 has a speaker and is also capable of outputting audio.

The input interface 4 is configured to receive operations to set a predetermined position (e.g., a region of interest), to set the display orientation of an image, to input numerical values, to switch between modes, and the like. For example, the input interface 4 is realized by using a trackball, a switch, a button, a mouse, a keyboard, a touchpad on which input operations are performed by touching the operation surface thereof, a touch monitor in which a display screen and a touchpad are integrally formed, a contactless input circuit using an optical sensor, an audio input circuit, and/or the like. The input interface 4 is connected to processing circuitry 55 (explained later) and is configured to convert the input operations received from the operator into electrical signals and to output the electrical signals to the processing circuitry 55. In the present disclosure, the input interface 4 does not necessarily have to include physical operation component parts such as a mouse, a keyboard, and/or the like. Possible examples of the input interface include an electric signal processing circuit configured to receive an electric signal corresponding to an input operation from an external input device provided separately from the apparatus and to output the received electric signal to the processing circuitry 55.

The apparatus main body 5 includes the transmission and reception circuitry 51, B-mode processing circuitry 52, Doppler processing circuitry 53, a memory 54, and the processing circuitry 55. In the ultrasound diagnosis apparatus 1 illustrated in FIG. 1, processing functions are stored in the memory 54 in the form of computer-executable programs. The transmission and reception circuitry 51, the B-mode processing circuitry 52, the Doppler processing circuitry 53, and the processing circuitry 55 are processors configured to realize the functions corresponding to the programs, by reading and executing the programs from the memory 54. In other words, the circuits that have read the programs have the functions corresponding to the read programs.

Each of the transmission and reception circuitry 51 includes a pulse generator, a transmission delay circuit, a pulser, and the like and is configured to supply the drive signal to the ultrasound probe 2. The pulse generator is configured to repeatedly generate a rate pulse for forming a transmission ultrasound wave at a predetermined rate frequency. Further, the transmission delay circuit is configured to apply a delay time period that is required to converge the ultrasound waves generated by the ultrasound probe 2 into the form of a beam and to determine transmission directionality and that corresponds to each of the piezoelectric transducer elements, to each of the rate pulses generated by the pulse generator. Also, the pulser is configured to apply the drive signal (a drive pulse) to the ultrasound probe 2 with timing based on the rate pulses. In this situation, the transmission and reception circuitry 51 according to the present embodiment are configured to supply the drive signal delayed for each of the element groups (groups of piezoelectric transducer elements) that are obtained by connecting the piezoelectric transducer elements divided in grid sections in a formation of multiple concentric circles. This aspect will be explained later.

Each of the transmission and reception circuitry 51 has a function that is able to instantly change the transmission frequency, the transmission drive voltage, and the like, for the purpose of executing a predetermined scan sequence according to instructions from the processing circuitry 55 (explained later). In particular, the function to change the transmission drive voltage is realized by using a linear-amplifier-type transmission circuit of which the value can be instantly switched or by using a mechanism configured to electrically switch between a plurality of power source units.

Further, each of the transmission and reception circuitry 51 includes a pre-amplifier, an Analog/Digital (A/D) converter, a reception delay circuit, an adder, and the like and is configured to generate reflected-wave data by performing various types of processes on the reflected-wave signals received by the ultrasound probe 2. The pre-amplifier is configured to amplify the reflected-wave signals in correspondence with channels. The A/D converter is configured to perform an A/D conversion on the amplified reflected-wave signals. The reception delay circuit is configured to apply delay time periods required to determine reception directionality. The adder is configured to generate the reflected-wave data by performing an adding process on the reflected-wave signals processed by the reception delay circuit. As a result of the adding process performed by the adder, reflected components of the reflected-wave signals that are from the direction corresponding to the reception directionality are emphasized, so that a comprehensive beam for ultrasound transmission and reception is formed according to the reception directionality and the transmission directionality. The transmission and reception circuitry 51 are an example of the driving circuits.

The B-mode processing circuitry 52 is configured to generate data (B-mode data) in which the signal intensity is expressed by a degree of brightness, by receiving the reflected-wave data from the transmission and reception circuitry 51 and performing a logarithmic amplification, an envelope detecting process, and/or the like thereon.

The Doppler processing circuitry 53 is configured to generate data (Doppler data) obtained by extracting moving member information such as velocity, dispersion, power, and the like with respect to multiple points, by performing a frequency analysis to obtain velocity information from the reflected-wave data received from the transmission and reception circuitry 51 and extracting a blood flow, a tissue, and a contrast agent echo component subject to the Doppler effect. The moving members in the present embodiment are fluids such as blood flowing in blood vessels, lymph flowing in lymphatic ducts, and the like.

In this situation, the B-mode processing circuitry 52 and the Doppler processing circuitry 53 are each capable of processing both two-dimensional reflected-wave data and three-dimensional reflected-wave data. In other words, the B-mode processing circuitry 52 is configured to generate two-dimensional B-mode data from two-dimensional reflected-wave data and to generate three-dimensional B-mode data from three-dimensional reflected-wave data. Further, the Doppler processing circuitry 53 is configured to generate two-dimensional Doppler data from two-dimensional reflected-wave data and to generate three-dimensional Doppler data from three-dimensional reflected-wave data. The three-dimensional B-mode data is data in which a brightness value is assigned in correspondence with the intensity of reflection from a reflection source positioned at each of a plurality of points (sampling points) set on scanning lines within a three-dimensional scanned range. Further, the three-dimensional Doppler data is data in which a brightness value is assigned in correspondence with a value of blood flow information (velocity, dispersion, and power) to each of a plurality of points (sampling points) set on the scanning lines within the three-dimensional scanned range.

The memory 54 is configured to store therein a display-purpose ultrasound image generated by the processing circuitry 55. Further, the memory 54 is also configured to store therein any of the data generated by the B-mode processing circuitry 52 and the Doppler processing circuitry 53. Further, the memory 54 is also configured to store therein control programs for performing the ultrasound transmission and reception, image processing processes, and display processes, as well as various types of data such as diagnosis information (e.g., patients' IDs, medical doctors' observations), diagnosis protocols, various types of body marks, and the like.

The processing circuitry 55 is configured to control the entirety of processes performed by the ultrasound diagnosis apparatus 1. More specifically, the processing circuitry 55 performs various types of processes by reading and executing programs corresponding to a controlling function 551, an image generating function 552, and a display controlling function 553 illustrated in FIG. 1, from the memory 54.

On the basis of the various types of setting requests input by the operator via the input interface 4 and the various types of control programs and the various types of data read from the memory 54, the controlling function 551 is configured to control processes performed the transmission and reception circuitry 51, the B-mode processing circuitry 52, and the Doppler processing circuitry 53.

The image generating function 552 is configured to generate ultrasound images from the data generated by the B-mode processing circuitry 52 and the Doppler processing circuitry 53. In other words, the image generating function 552 is configured to generate B-mode image data in which the intensities of the reflected waves are expressed as brightness levels, from the two-dimensional B-mode data generated by the B-mode processing circuitry 52. The B-mode image data is data rendering the shape of a tissue in the region subject to an ultrasound scan. Further, the image generating function 552 is configured to generate Doppler image data indicating the moving member information, from the two-dimensional Doppler data generated by the Doppler processing circuitry 53. The Doppler image data is velocity image data, dispersion image data, power image data, or image data combining together any of these types of image data. The Doppler image data is data indicating fluid information related to the fluid flowing through the region subject to an ultrasound scan.

In this situation, generally speaking, the image generating function 552 converts (by performing a scan convert process) a scanning line signal sequence from an ultrasound scan into a scanning line signal sequence in a video format used by, for example, television and generates the display-purpose ultrasound image. More specifically, the image generating function 552 generates the display-purpose ultrasound image by performing a coordinate transformation process compliant with the ultrasound scanning mode used by the ultrasound probe 2.

Further, as various types of image processing processes besides the scan convert process, the image generating function 552 performs, for example, an image processing process (a smoothing process) to re-generate an average brightness value image, an image processing process (an edge enhancement process) that uses a differential filter inside an image, or the like, by using a plurality of image frames resulting from the scan convert process. Also, the image generating function 552 combines text information of various types of parameters, scale graduations, body marks, and the like with the ultrasound image.

In other words, the B-mode data and the Doppler data are each ultrasound image data before the scan convert process. The data generated by the image generating function 552 is the display-purpose ultrasound image after the scan convert process. The B-mode data and the Doppler data may be referred to as raw data.

Further, the image generating function 552 is also capable of generating three-dimensional B-mode image data by performing a coordinate transformation process on the three-dimensional B-mode data generated by the B-mode processing circuitry 52. Further, the image generating function 552 is also capable of generating three-dimensional Doppler image data by performing a coordinate transformation process on the three-dimensional Doppler data generated by the Doppler processing circuitry 53. In other words, the image generating function 552 is also capable of generating "the three-dimensional B-mode image data and the three-dimensional Doppler image data" as "volume data represented by three-dimensional ultrasound image data".

Further, the image generating function 552 is also capable of performing a rendering process on the volume data, for the purpose of generating various types of two-dimensional image data used for displaying the volume data on the display 3.

The display controlling function 553 is configured to exercise control so that the display 3 displays the display-purpose ultrasound image stored in the memory 54. Further, the controlling function 551 is configured to exercise control so that the display 3 displays processing results obtained by the functions.

An overall configuration of the ultrasound diagnosis apparatus 1 according to the first embodiment has thus been explained. The ultrasound diagnosis apparatus 1 according to the first embodiment configured as described above includes an ultrasound probe in which a transducer element array divided in grid sections is connected in a formation of multiple concentric circles with excellent area efficiency. As explained above, in two-dimensional array probes, because a driving circuit is provided in the vicinity of piezoelectric transducer elements so as to control the driving of the piezoelectric transducer elements, scans have a high degree of freedom, which makes three-dimensional scans possible. However, compared to when being driven by the apparatus main body, two-dimensional array probes have lower transmission/reception capabilities due to restrictions of the area and electric power consumption of an Integrated Circuit (IC) installed in the ultrasound probe. Accordingly, there are restrictions in Signal-to-Noise (S/N) ratios, dynamic ranges, bandwidths, and the like, which make it difficult to acquire high-quality cross-sectional images.

Further, although the abovementioned annular array is capable of acquiring high-quality cross-sectional images, because the annular array is formed by providing the transducer element array divided in the grid sections with the electrodes in the formation of the multiple concentric circles, a problem may arise where an effective area in the transducer element array is decreased and sidelobes increase, because it is necessary to provide a gap equal to or larger than one element between the electrodes arranged in the concentric circular formation, for the purpose of acoustically and electrically separating the channels from one another.

Further, generally speaking, annular arrays are structured in such a manner that the area of the transducer elements included in each of the channels in the concentric circular formation is constant, so as to achieve uniform characteristics by keeping the load (transducer element capacitance) on the driving circuit constant among the channels. Accordingly, in annular arrays, the radius pitch between the channels changes with less finely in a central part and more finely in a peripheral part. For this reason, in the central part, the control on the aperture and delays per channel is exercised less finely, and the image quality is therefore degraded at short distances for which transmission and reception is performed with a smaller aperture. In contrast, in the peripheral part, the proportion of the gaps between the electrodes is larger, which is inefficient.

To cope with the circumstances described above, the present disclosure realizes an annular array in which a transducer element array divided in grid sections is connected in a formation of multiple concentric circles with excellent area efficiency, by using a substrate provided with wirings configured to cause electrodes for a plurality of piezoelectric transducer elements divided in a grid sections to be connected in parallel in a formation of substantially concentric circles. Further, in the present disclosure, with the above configuration, it is possible to make the radius pitch in the central part fine. Accordingly, it is possible to realize an excellent beam form even at short distances for which transmission and reception is performed with a smaller aperture. It is therefore possible to improve image quality for the short distances.

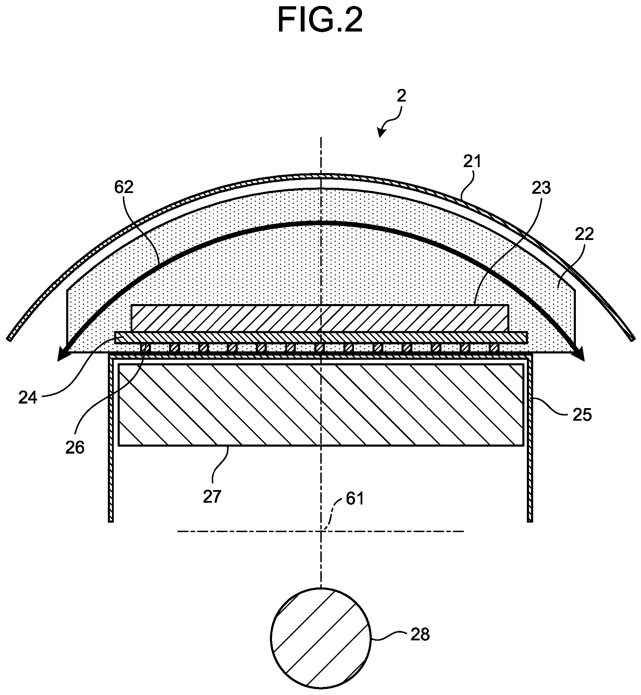

Details of the ultrasound probe 2 according to the first embodiment will be explained below. FIG. 2 is a drawing illustrating an exemplary configuration of the ultrasound probe 2 according to the first embodiment. FIG. 2 presents a cross-sectional view of the ultrasound probe 2. Further, FIG. 2 illustrates the example in which Flexible Preprint Circuits (FPCs) are used as the substrate provided with the wirings configured to cause the electrodes for the plurality of piezoelectric transducer elements divided in the grid sections to be connected in parallel in the formation of the substantially concentric circles.

As illustrated in FIG. 2, the ultrasound probe 2 includes a window member 21, an acoustic matching layer 22, piezoelectric transducer elements 23, a first FPC 24, a second FPC 25, bumps 26, a backing member (backing) 27, and a motor 28.

For example, the piezoelectric transducer elements 23 have electrodes being structured with Lead Zirconate Titanate (PZT) or the like and facing the acoustic emission surface side (the top side of the drawing) and the rear surface side (the bottom side of the drawing) and is configured to transmit and receive ultrasound waves under the control of the transmission and reception circuitry 51. In the ultrasound probe 2 according to the present embodiment, the piezoelectric transducer elements 23 are divided in grid sections, so as to form a plurality of transducer elements arranged in a two-dimensional matrix formation. In addition, further provided on the acoustic emission surface side of the piezoelectric transducer elements 23 are the acoustic matching layer 22 configured to mitigate mismatching of acoustic impedances between the piezoelectric transducer elements and the subject; and the window member 21 configured to come into contact with the subject.

The first FPC 24 is laid on the rear surface side of the piezoelectric transducer elements 23. By using a conductive pattern provided on the side different from the piezoelectric transducer elements 23 side, the first FPC 24 is configured to cause a plurality of electrodes positioned close to the center of the piezoelectric transducer elements 23 to be connected in parallel in a formation of substantially concentric circles. More specifically, the first FPC 24 is provided, on the rear surface side, with the conductive pattern that causes a group of transducer elements in a prescribed quantity positioned at similar distances from the array center of the piezoelectric transducer elements 23 among the transducer elements in the grid formation of the piezoelectric transducer elements 23 to be connected in parallel. In other words, the first FPC 24 is provided, on the rear surface side, with the conductive pattern in the formation of the substantially concentric circles.

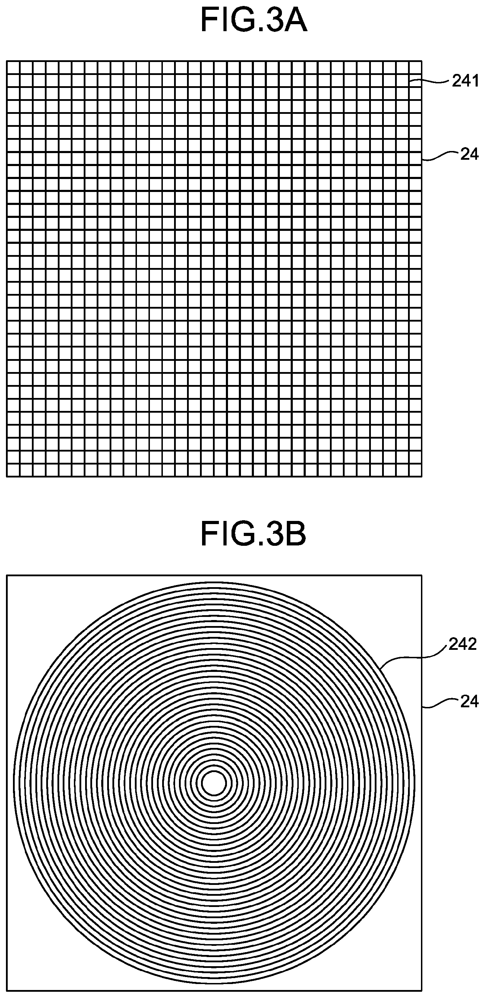

FIGS. 3A and 3B are drawings for explaining the first FPC 24 according to the first embodiment. FIG. 3A illustrates the surface of the first FPC on the piezoelectric transducer elements 23 side. FIG. 3B illustrates the surface on the rear surface side. For example, as illustrated in FIG. 3A, the first FPC 24 has a grid-like conductive pattern 241 provided on the surface on the piezoelectric transducer elements 23 side. The conductive pattern 241 corresponds to the transducer elements arranged in the grid formation of the piezoelectric transducer elements 23 and is joined with electrodes for the transducer elements.

Further, for example, as illustrated in FIG. 3B, the first FPC 24 has a conductive pattern 242 in a formation of substantially concentric circles provided on the surface on the rear surface side. By using a plurality of wirings in annular formations, the conductive pattern 242 causes a plurality of electrodes corresponding to the transducer elements in the piezoelectric transducer elements 23 to be connected in parallel in the formation of the substantially concentric circles. In other words, by using each annular wiring, the conductive pattern 242 connects a plurality of electrodes in parallel, so that the transducer elements corresponding to the electrodes connected in parallel form one transducer element group. In one example, the wiring positioned outermost in the conductive pattern 242 illustrated in FIG. 3B forms one transducer element group, by causing the electrodes for the plurality of transducer elements in corresponding positions on the piezoelectric transducer elements 23 side to be connected in parallel. In the ultrasound probe 2 according to the present embodiment, the one transducer element group described above corresponds to one channel.

Similarly, the annular wirings arranged in the formation of the substantially concentric circles form transducer element groups, by each causing electrodes for a plurality of transducer elements in corresponding positions on the piezoelectric transducer elements 23 side to be connected in parallel. Although FIG. 3B illustrates the annular wirings by using thin-lined circles, the wirings have certain thicknesses in actuality to cause the grid-forming transducer elements to be connected in parallel in the annular formation and may be in annular forms that are not circles.

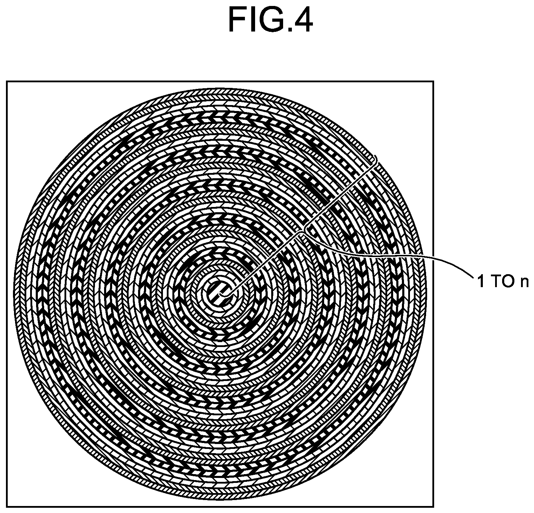

In this situation, as illustrated in FIG. 3B, as a result of the conductive pattern 242 of the ultrasound probe 2 forming the plurality of wirings of which the radius pitch from the center is fine, it is possible to realize an annular array having a fine radius pitch between the channels. FIG. 4 is a drawing for explaining the channels of the ultrasound probe 2 according to the first embodiment. In FIG. 4, the plurality of transducer elements contained in each of the regions indicated with the same concentric circular hatching structure one transducer element group, i.e., one channel. For example, as for the channels of the ultrasound probe 2, the abovementioned conductive pattern 242 forms as many channels as n, from the center toward the outside, as illustrated in FIG. 4.

Further, as illustrated in FIG. 4, the ultrasound probe 2 is configured so that the radius pitch is substantially constant between adjacently-positioned channels. In other words, the conductive pattern 242 of the first FPC 24 causes a plurality of transducer elements positioned at similar distances from the center of the transducer element array to be connected in parallel, so that the radius pitch is substantially constant between adjacently-positioned channels.

As explained above, in the ultrasound probe 2 according to the present embodiment, because the electrodes corresponding to the transducer elements are connected in parallel in the formation of the substantially concentric circles on the rear surface side of the first FPC 24, it is possible to utilize all the transducer elements inside the conductive pattern 242 for the ultrasound transmission and reception, without the need to purposefully provide gaps for acoustically and electrically separating the channels from one another. It is therefore possible to cause the transducer element array divided in the grid sections to be connected in the formation of the multiple concentric circles with excellent area efficiency.

Returning to the description of FIG. 2, the second FPC 25 is laid on the rear surface of the first FPC 24, has conductive parts in positions corresponding to the positions of the conductive parts of the first FPC 24, and is configured to transmit and receive signals to and from the first FPC 24. More specifically, the second FPC 25 is an Input/Output (I/O)-purpose substrate laid on the conductive pattern 242 side of the first FPC 24. The second FPC 25 is connected to a signal line included in the cable used for the connection to the apparatus main body 5.

The bumps 26 are configured to electrically connect the first FPC 24 and the second FPC 25 to each other. In this situation, the first FPC 24 and the second FPC 25 are formed so that the exposed positions of the conductive parts are aligned. In other words, when the first FPC 24 and the second FPC 25 are placed on top of each other, the exposed positions of the conductive parts of these FPCs are determined so that the exposed positions of the conductive pattern 242 of the first FPC 24 correspond to the exposed positions of the conductive parts of the second FPC 25.

In this situation, the conductive parts (the exposed positions) of the conductive pattern 242 of the first FPC 24 are provided for each of the wirings corresponding to the channels. In one example, the conductive parts are provided for each of the n wirings corresponding to channels 1 to n illustrated in FIG. 4. In this situation, as for the conductive parts (the exposed positions) of the first FPC 24, the quantity and the positions thereof are arbitrary, as long as an impedance is ensured to allow a sufficient electric current to flow.

The bumps 26 are arranged between the exposed positions of the conductive pattern 242 of the first FPC 24 and the exposed positions of the conductive parts of the second FPC 25 and are configured to electrically connect the first FPC 24 and the second FPC 25 to each other. In this situation, the gaps between the first FPC 24 and the second FPC 25 other than the bumps 26 are filled with a non-conducive adhesive agent.

The motor 28 is configured to swing the piezoelectric transducer elements 23 under control of the controlling function 551. More specifically, the motor 28 swings the plurality of piezoelectric transducer elements divided in the grid sections and the substrate. For example, on the basis of a control signal received from the controlling function 551, the motor 28 swings the acoustic matching layer 22, the piezoelectric transducer elements 23, the first FPC, the second FPC 25, the bumps 26, and the backing member 27, in the directions indicated by an arrow 62, while using an intersection point 61 illustrated in FIG. 2 as a rotation axis. The motor 28 is an example of the swinging unit.

With annular arrays, it would be difficult to deflect ultrasound beams in a scanning direction by controlling and driving piezoelectric transducer elements. Accordingly, the ultrasound probe 2 includes the motor 28, so as to perform a scan with the swinging caused by the motor 28.

As explained above, the ultrasound probe 2 realizes an annular array in which the transducer element array divided in the grid sections is connected in the formation of the multiple concentric circles with excellent area efficiency, by providing, on the rear surface side of the first FPC 24, the wirings configured to cause the electrodes for the plurality of piezoelectric transducer elements divided in the grid sections to be connected in parallel in the formation of the substantially concentric circles.

Further, for the purpose of realizing uniform characteristics by keeping the loads (transducer element capacitance) on the transmission and reception circuitry 51 driving the channels substantially constant, the ultrasound probe 2 according to the present embodiment is configured so as to keep the ratio between the quantity of the transmission and reception circuitry 51 driving each of the channels and the quantity of the transducer elements included in the corresponding transducer element group substantially constant.

More specifically, each of the transducer element groups corresponding to the electrodes connected in parallel in the formation of the substantially concentric circles is connected in parallel to one or more transmission and reception circuitry 51. Among the transducer element groups each connected in parallel to the one or more transmission and reception circuitry 51, the ratio between the quantity of the transducer elements and the quantity of the transmission and reception circuits is kept substantially constant. In other words, a transducer element group including fewer transducer elements is driven by fewer transmission and reception circuitry 51. Conversely, a transducer element group including more transducer elements is driven by more transmission and reception circuitry 51.

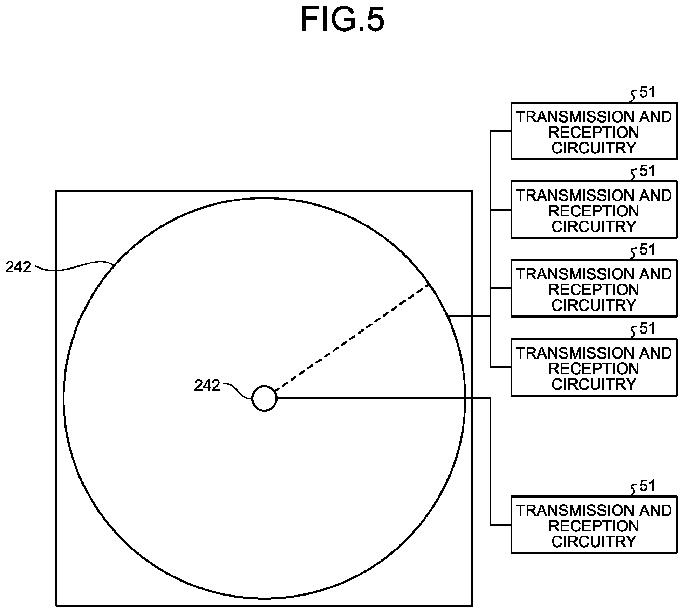

FIG. 5 is a drawing for explaining connections to the transmission and reception circuitry 51 from the ultrasound probe 2 according to the first embodiment. As the conductive pattern 242 connected to the transmission and reception circuitry 51, FIG. 5 illustrates only two wirings, namely, the innermost wiring and the outermost wiring, for the sake of convenience in the explanation; however, in actuality, a plurality of other wirings are respectively connected to one or more transmission and reception circuitry 51. For example, as illustrated in FIG. 5, in the ultrasound probe 2, the transducer element group connected in parallel by the innermost wiring includes fewer transducer elements and is driven by a single transmission and reception circuitry 51. In contrast, the transducer element group connected in parallel by the outermost wiring includes more transducer elements and is driven by four transmission and reception circuitry 51, for example.

As explained above, each of the transducer element groups in the ultrasound probe 2 is connected in parallel to one or more transmission and reception circuitry 51 in a quantity corresponding to the quantity of the included transducer elements and is driven by the one or more transmission and reception circuitry 51 connected in parallel. In this situation, by keeping the ratio between the quantity of the transducer elements and the quantity of the transmission and reception circuits substantially constant among the transducer element groups, it is possible to realize uniform characteristics by keeping the loads (the transducer element capacitance) on the transmission and reception circuitry 51 driving the channels substantially constant.

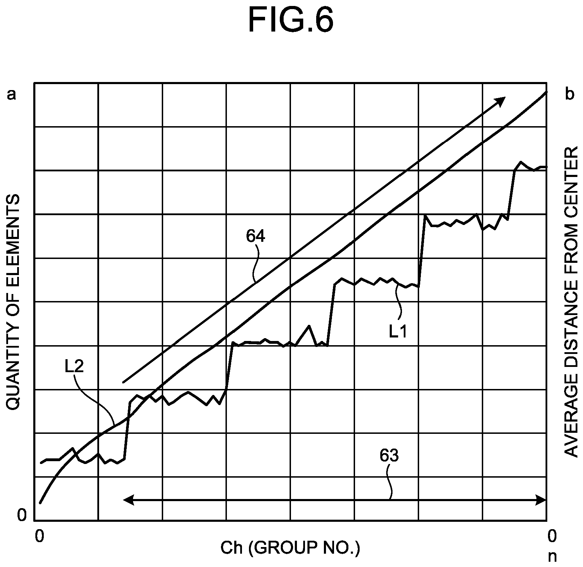

FIG. 6 is a chart illustrating a relationship between the quantity of transducer elements and average distances in the ultrasound probe 2 according to the first embodiment. In FIG. 6, the vertical axis expresses the quantity of elements (the quantity of the transducer elements) and average distances from the center of the transducer element array, whereas the horizontal axis expresses the channels (group numbers [Nos.]). Further, in FIG. 6, the line L1 indicates the quantity of elements for each of the channels, whereas the line L2 indicates an average distance from the center of the transducer elements with respect to each of the channels.

For example, in the ultrasound probe 2, as indicated by the line L1 in FIG. 6, the quantity of the transducer elements is varied for each channel. In this situation, in the ultrasound probe 2, the quantity of the transducer elements included for each of the channels is varied discretely as indicated by the line L1, to facilitate the correspondence with the quantity of the transmission and reception circuitry 51. In other words, in the ultrasound probe 2, there are two or more channels including an equal number of transducer elements, and the quantity of the included transducer elements is discreetly increased.

In this situation, each of the channels is connected in parallel to one or more transmission and reception circuitry 51 in a quantity corresponding to the quantity of the included transducer elements. For example, in the ultrasound probe 2 illustrated in FIG. 6, to each of the channels included in the range indicated by an arrow 63, two or more transmission and reception circuitry 51 are connected in parallel. In contrast, to each of the channels outside the range indicated by the arrow 63 (i.e., from 0 to the left end of the arrow 63), one transmission and reception circuitry 51 is connected. For each of the channels included in the range indicated by the arrow 63, the more transducer elements the channel has, the more transmission and reception circuitry 51 are connected in parallel.

In the ultrasound probe 2 configured as described above, the average distance from the center for the transducer elements corresponding to each of the channels linearly changes, as indicated by the line L2 in FIG. 6. In particular, in the range indicated by an arrow 64 in FIG. 6 (i.e., the channels to each of which two or more transmission and reception circuitry 51 are connected), the average distance from the center for the transducer elements included in each transducer element group changes more linearly. In other words, for the transducer element groups each connected in parallel to two or more transmission and reception circuitry 51, the average distances from the center are kept at substantially constant intervals. It is therefore possible to control apertures and delays with a high level of precision.

As explained above, the ultrasound probe 2 is configured with the annular array realizing uniform characteristics, by causing the transducer element array divided in the grid sections to be connected in the formation of the multiple concentric circles with excellent area efficiency and keeping the loads (the transducer element capacitance) on the transmission and reception circuitry 51 driving the channels substantially constant. In the explanations above, the example was explained in which a cross-section is scanned by swinging the piezoelectric transducer elements 23 and the like by using the built-in motor 28; however, possible embodiments are not limited to this example. For instance, it is also acceptable to scan a cross-section by changing the position and the orientation of the ultrasound probe 2 by using a mechanism provided externally.

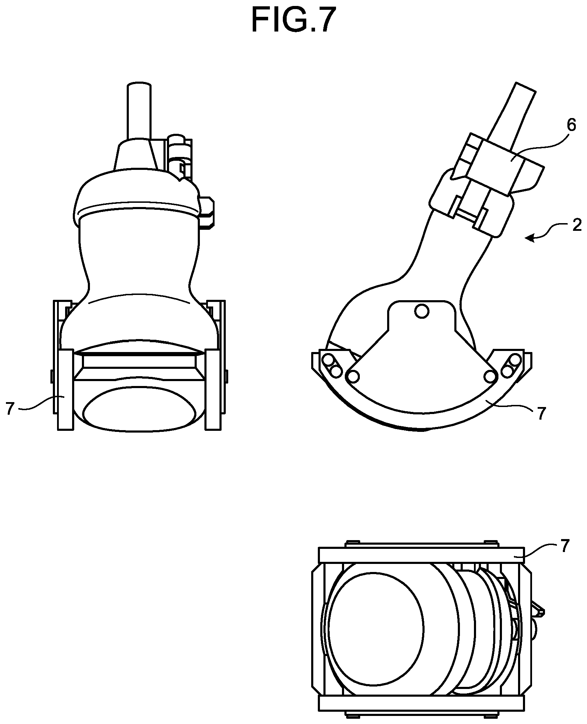

FIG. 7 is a drawing illustrating an example of a swinging mechanism according to the first embodiment. FIG. 7 illustrates an example in which a swinging mechanism 7 is provided externally to the ultrasound probe 2. Further, FIG. 7 illustrates the example in which the swinging mechanism 7 to which the ultrasound probe 2 is attached is viewed in the swinging direction of the ultrasound probe 2 (top left), in a direction orthogonal to the swinging direction (top right), and from the transmission and reception surface side of the ultrasound probe 2 (bottom).

For example, as illustrated in FIG. 7, the swinging mechanism 7 is configured so that the surface to be in contact with the subject has the same curvature radius as that of the transmission and reception surface of the ultrasound probe 2 and, when being attached to the ultrasound probe 2, is at the same height as that of the transmission and reception surface. Further, the swinging mechanism 7 has a supporting mechanism that supports the attached ultrasound probe 2. In other words, the ultrasound probe 2 is rotatably and pivotally supported by the supporting mechanism. The operator is able to change the direction of the ultrasound beam by tilting the ultrasound probe 2 in directions orthogonal to the supporting mechanism, while gripping the ultrasound probe 2.

In this situation, a position sensor 6 is attached to the ultrasound probe 2 illustrated in FIG. 7. In other words, for manual scans performed by the operator, the position sensor 6 is used to bring data into correspondence with acquisition positions. In that situation, for example, the image generating function 552 obtains position information from the position sensor 6. Further, on the basis of time information, the image generating function 552 stores the position information obtained by the position sensor 6 into the memory 54 so as to be kept in correspondence with the data generated by the B-mode processing circuitry 52 and the Doppler processing circuitry 53.

In other words, on the basis of acquisition times of the reflected-wave data and times at which the position information was obtained by the position sensor 6, the image generating function 552 brings the position information into correspondence with the data. As a result, the image generating function 552 is able to generate accurate ultrasound images from the manual scans. In this situation, the position sensor 6 may be, for example, a magnetic sensor configured to obtain the position information by detecting a three-dimensional magnetic field formed by a transmitter.

Further, in the explanation above, the example was explained in which the ultrasound probe 2 is used in the manual scans performed by the operator; however, possible embodiments are not limited to this example. For instance, the ultrasound probe 2 may be held by a robot arm and used in a scan under control of the robot. In that situation, for example, the image generating function 552 sets a predetermined position of the ultrasound probe 2 held by the robot arm as an initial position. Further, an obtaining function 162 obtains a moving amount from the initial position of the robot arm holing the ultrasound probe 2, as displacement amounts of the position and the orientation of the ultrasound probe 2, so that the data and the position information are brought into correspondence with each other on the basis of the obtained displacement amounts.

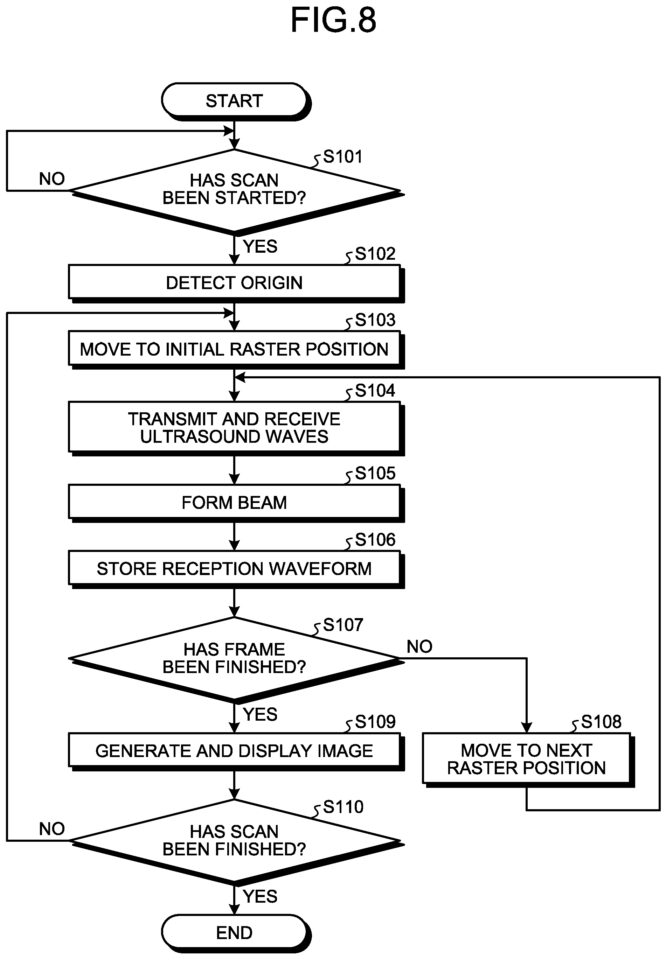

Next, a process performed by the ultrasound diagnosis apparatus 1 according to the first embodiment will be explained, with reference to FIG. 8. FIG. 8 is a flowchart for explaining a procedure in the process performed by the ultrasound diagnosis apparatus 1 according to the first embodiment. FIG. 8 illustrates an example in which a scan is performed by the motor 28. Further, steps S101 through S110 are steps realized as a result of the processing circuitry 55 reading and executing the programs corresponding to the controlling function 551, the image generating function 552, the display controlling function 553, from the memory 54.

As illustrated in FIG. 8, in the ultrasound diagnosis apparatus 1 according to the first embodiment, the processing circuitry 55 judges whether a scan has been started (step S101). When a scan has been started (step S101: Yes), the processing circuitry 55 detects the position of the origin of the piezoelectric transducer elements 23 and the like (step S102) and moves the piezoelectric transducer elements 23 and the like to an initial raster position, by controlling the motor 28 on the basis of the detected position of the origin (step S103). Unless a scan has been started (step S101: No), the processing circuitry 55 is in a standby state.

Further, by controlling the transmission and reception circuitry 51 so as to transmit and receive ultrasound waves (step S104), the processing circuitry 55 causes a beam to be formed (step S105) and stores a reception waveform into the memory 54 (step S106). After that, the processing circuitry 55 judges whether the acquisition of the reception waveform corresponding to one frame has been finished (step S107). When the acquisition of the reception waveform corresponding to one frame has not been finished (step S107: No), the processing circuitry 55 controls the motor 28 so as to move the piezoelectric transducer elements 23 and the like to the next raster position (step S108) and continues with the processes at step S104 and thereafter.

On the contrary, at step S107, when the acquisition of the reception waveform corresponding to one frame has been finished (step S107: Yes), the processing circuitry 55 generates and displays an ultrasound image (step S109) and judges whether the scan has been finished (step S110). When the scan has not been finished (step S110: No), the processing circuitry 55 returns to step S103 and continues the process. On the contrary, when the scan has been finished (step S110: Yes), the processing circuitry 55 ends the process.

As explained above, according to the first embodiment, the piezoelectric transducer elements 23 are divided in the grid sections. The substrate is laid on the rear surface of the plurality of piezoelectric transducer elements and has the wirings configured to cause the plurality of electrodes for the plurality of piezoelectric transducer elements to be connected in parallel in the formation of the substantially concentric circles. Accordingly, the ultrasound probe 2 according to the first embodiment is able to utilize all the transducer elements inside the conductive pattern 242 for the ultrasound transmission and reception, without the need to purposefully provide gaps for acoustically and electrically separating the channels from one another. It is therefore possible to realize the annular array in which the transducer element array divided in the grid sections is connected in the formation of the multiple concentric circles with excellent area efficiency. Further, the ultrasound probe 2 according to the first embodiment makes it possible to realize an annular array by which sidelobes are not increased. Further, the ultrasound probe 2 according to the first embodiment is able to keep small the radii of the transducer element groups in the central part of the piezoelectric transducer elements 23 and the radius pitch in the central part. Accordingly, it is possible to realize an excellent beam form even at short distances for which the transmission and reception is performed with a smaller aperture and to obtain ultrasound images having high image quality.

Further, according to the first embodiment, the substrate is the first FPC 24 configured to cause the plurality of electrodes positioned close to the center of the piezoelectric transducer elements to be connected in parallel in the formation of the substantially concentric circles, by using the conductive pattern provided on the side different from the plurality of piezoelectric transducer elements side. Accordingly, the ultrasound probe 2 according to the first embodiment makes it possible to easily realize the annular array in which the transducer element array divided in the grid sections is connected in the formation of the multiple concentric circles with excellent area efficiency. Further, as the transducer elements, the ultrasound probe 2 according to the first embodiment is able to use a ferroelectric material such as PZT. It is therefore possible to increase electroacoustic conversion efficiency and to improve sensitivity, compared to annular arrays using a polymeric piezoelectric material such as polyvinylidene difluoride (PVDF) .

Further, according to the first embodiment, in addition to the first FPC 24 serving as the substrate, further provided is the second FPC 25 that is laid on the rear surface of the first FPC 24, has the conductive parts in the positions corresponding to the positions of the conductive parts of the first FPC 24, and is configured to transmit and receive the signals to and from the first FPC 24. Between the first FPC 24 and the second FPC 25, the conductive parts are made conductive with each other via the conductive joint, while the gaps other than the conductively joined positions are filled with the non-conductive adhesive agent. Consequently, the ultrasound probe 2 according to the first embodiment makes it possible to easily realize a signal lead-out structure.

Further, according to the first embodiment, the transducer element groups corresponding to the electrodes connected in parallel to the formation of the substantially concentric circles are each connected in parallel to one or more transmission and reception circuitry 51. Among the transducer element groups each connected in parallel to the one or more transmission and reception circuitry 51, the ratio between the quantity of the transducer elements and the quantity of the transmission and reception circuits is kept substantially constant. Consequently, the ultrasound probe 2 according to the first embodiment is able to keep the loads (the transducer element capacitance) on the transmission and reception circuitry 51 substantially constant and thus makes it possible to realize uniform characteristics.

Further, according to the first embodiment, for the transducer element groups each connected in parallel to two or more transmission and reception circuitry 51, the average distances from the center are kept at the substantially constant intervals. Consequently, in the ultrasound probe 2 according to the first embodiment, it is possible to keep the radius pitch substantially constant, up to the outer circumferential part of the piezoelectric transducer elements 23. It is therefore possible to realize the ring-shaped wirings only on the rear surface of the FPC and to thus simplify the structure.

Further, according to the first embodiment, the motor 28 is configured to swing the piezoelectric transducer elements 23 divided in the grid sections and the substrate. Consequently, the ultrasound probe 2 according to the first embodiment makes it possible to scan a cross-section.

Further, according to the first embodiment, the swinging mechanism 7 is configured to swing the ultrasound probe 2.

Consequently, the ultrasound probe 2 according to the first embodiment makes it possible to scan a cross-section, without the need to have a mechanical swinging mechanism such as the motor 28 built therein.

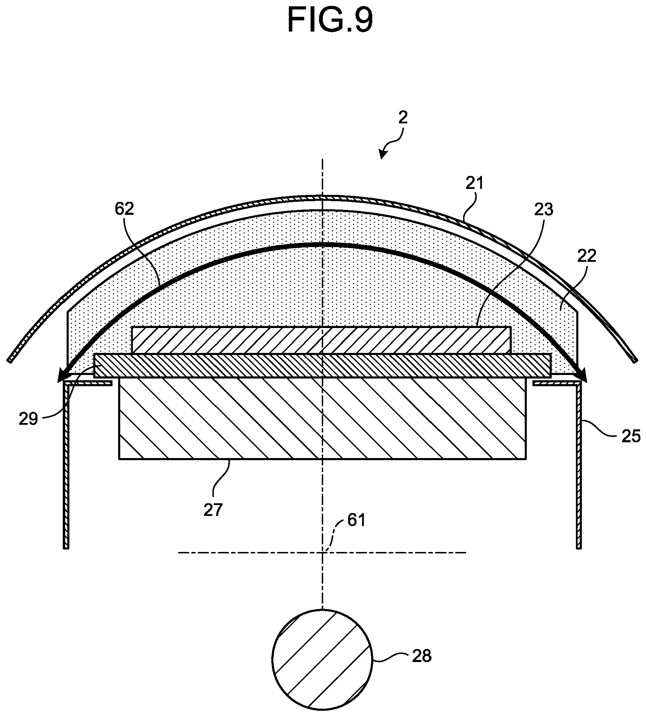

Second Example In the first embodiment, the example was explained in which the FPCs are used as the substrate. In a second example, which does not form part of the claimed invention, an example using a multi-layer substrate as the substrate will be explained. FIG. 9 is a drawing illustrating an exemplary configuration of an ultrasound probe according to the second example.

FIG. 9 illustrates a cross-sectional view of the ultrasound probe 2. Further, the ultrasound probe 2 according to the second example is different from that of the first embodiment for using the multi-layer substrate as the substrate. The second example will be explained below with a focus on the difference.

As illustrated in FIG. 9, the ultrasound probe 2 according to the second example includes the window member 21, the acoustic matching layer 22, the piezoelectric transducer elements 23, the second FPC 25, the backing member (the backing) 27, the motor 28, and a multi-layer substrate 29.

By using wirings mediated by a plurality of layers, the multi-layer substrate 29 is configured to cause a plurality of electrodes positioned closed to the center of the piezoelectric transducer elements 23 to be connected in parallel in a formation of substantially concentric circles. For example, the multi-layer substrate 29 has, on the outer surface on the acoustic emission surface side (the top side of the drawing), conductive parts corresponding to the grid-forming transducer elements of the piezoelectric transducer elements 23 and being joined with the electrodes for the transducer elements. Further, by using connections mediated by an inner layer and an outer layer on the rear surface side, the multi-layer substrate 29 causes the plurality of electrodes for the piezoelectric transducer elements 23 to be connected in parallel in the formation of the substantially concentric circles.

Although FIG. 9 illustrates the example in which a scan is performed while the motor 28 swings the piezoelectric transducer elements 23 and the like, it is also possible for the ultrasound probe 2 according to the second example to perform a scan with swinging caused by the swinging mechanism 7 or a robot arm, similarly to the first embodiment.

As explained above, according to the second example, the substrate is the multi-layer substrate 29 configured to cause the plurality of electrodes positioned close to the center of the piezoelectric transducer elements 23 to be connected in parallel in the formation of the substantially concentric circles, by using the wirings mediated by the plurality of layers. Consequently, the ultrasound probe 2 according to the second example makes it possible to realize a structure using the multi-layer substrate.

### Other Embodiments

The first embodiment, which is an embodiment falling within the scope of the claimed invention, has been explained. It is, however, possible to carry out the present disclosure in various different modes other than those in the first embodiment.

FIG. 1 illustrates the example in which the abovementioned processing functions are realized by the single processing circuit, namely, the processing circuitry 55; however, possible embodiments are not limited to this example. For instance, it is also possible to structure the processing circuitry 55 by combining together a plurality of independent processors, so that the processing functions are realized as a result of the processors executing the programs. Further, the processing functions of the processing circuitry 55 may be realized as distributed among or integrated into one or more processing circuits, as appropriate.

The term "processor" used in the above explanations denotes, for example, a Central Processing Unit (CPU), a Graphics Processing Unit (GPU), or a circuit such as an Application Specific Integrated Circuit (ASIC) or a programmable logic device (e.g., a Simple Programmable Logic Device [SPLD], a Complex Programmable Logic Device [CPLD], or a Field Programmable Gate Array [FPGA]). The processors realize the functions by reading and executing the programs saved in a memory. In this situation, instead of saving the programs in the memory, it is also acceptable to directly incorporate the programs in the circuits of the processors. In that situation, the processors realize the functions by reading and executing the programs incorporated in the circuits thereof. Further, the processors in the present embodiments do not each necessarily have to be structured as a single circuit. It is also acceptable to structure one processor by combining together a plurality of independent circuits so as to realize the functions thereof.

Further, the constituent elements of the apparatuses and the devices presented in the drawings for explaining the embodiments are based on functional concepts. Thus, it is not necessary to physically configure the constituent elements as indicated in the drawings. In other words, specific modes of distribution and integration of the apparatuses and the devices are not limited to those illustrated in the drawings. It is acceptable to functionally or physically distribute or integrate all or a part of the apparatuses and the devices in any arbitrary units, depending on various loads and the status of use. Further, all or an arbitrary part of the processing functions performed by the apparatuses and the devices may be realized by a CPU and a program analyzed and executed by the CPU or may be realized as hardware using wired logic.

As explained above, according to at least one aspect of the present embodiments, it is possible to cause the transducer element array divided in the grid sections to be connected in the formation of the multiple concentric circles with excellent area efficiency.

While certain embodiments have been described, these embodiments have been presented by way of example only, and are not intended to limit the scope of the inventions.

Various changes in the form of the embodiments described herein may be made without departing from the scope of the invention as defined by the appended claims.

## Claims

1. An ultrasound probe (2) comprising:
a plurality of piezoelectric transducer elements (23) divided in grid sections,
the piezoelectric transducer elements having an ultrasound wave transmitting/receiving surface and a rear surface opposite to the ultrasound wave transmitting/receiving surface;
the piezoelectric transducer elements (23) having electrodes facing the ultrasound wave transmitting/receiving surface side and the rear surface side;
and
a substrate (24) provided on the rear surface of the plurality of piezoelectric transducer elements (23),
**characterized in that**
the substrate (24) is a Flexible Preprint Circuit, FPC, having a plurality of wirings in annular formations on a side different from the plurality of piezoelectric transducer elements (23) side and that connect a plurality of electrodes corresponding to the transducer elements in the plurality of piezoelectric transducer elements (23) in parallel in the formation of substantially concentric circles, the plurality of wirings in annular formations connecting the plurality of electrodes to driving circuity.

2. The ultrasound probe (2) according to claim 1, further comprising, in addition to a first FPC (24) serving as the substrate, a second FPC (25) that is provided on a rear surface of the first FPC (24), has a conductive part in a position corresponding to a position of a conductive part of the first FPC (24), and is configured to transmit and receive a signal to and from the first FPC (24), wherein
between the first FPC (24) and the second FPC (25), the conductive parts are made conductive with each other via a conductive joint, while gaps other than conductively joined positions are filled with a non-conductive adhesive agent.

3. The ultrasound probe (2) according to any one of claims 1 to 2, wherein each of groups of piezoelectric transducer elements (23) corresponding to electrodes located in substantially concentric circles is connected in parallel to one or more driving circuitry.

4. The ultrasound probe (2) according to claim 3, wherein,
among the groups of piezoelectric transducer elements, a ratio between a quantity of piezoelectric transducer elements and a quantity of driving circuits is kept substantially constant.

5. The ultrasound probe (2) according to claims 3 or 4, wherein, for groups of piezoelectric transducer elements (23) each connected to two or more driving circuits, average distances from a center of the plurality of piezoelectric transducer elements are kept at substantially constant intervals.

6. The ultrasound probe (2) according to any one of claims 1 to 5, further comprising a swinging unit (28) configured to swing the plurality of piezoelectric transducer elements (23) divided in the grid sections and the substrate (24, 29).

7. An ultrasound diagnosis apparatus (1) comprising:
an ultrasound probe (2) according to any one of claims 1 to 6; and
a generating unit (552) configured to generate an ultrasound image on a basis of a signal received by the ultrasound probe (2).

8. The ultrasound diagnosis apparatus (1) according to claim 7, further comprising: a swinging mechanism (7) configured to swing the ultrasound probe (2).

## Patentansprüche

1. Ultraschallsonde (2), umfassend:
eine Vielzahl von piezoelektrischen Wandlerelementen (23), die in Rasterabschnitte unterteilt ist, wobei die piezoelektrischen Wandlerelemente eine Ultraschallwellen-Sende-/Empfangsfläche und eine der Ultraschallwellen-Sende-/Empfangsfläche gegenüberliegende Rückfläche aufweisen;
wobei die piezoelektrischen Wandlerelemente (23) Elektroden aufweisen, die der Ultraschallwellen-Sende-/Empfangsflächenseite und der Rückflächenseite zugewandt sind; und
ein Substrat (24), das auf der Rückfläche der Vielzahl von piezoelektrischen Wandlerelementen (23) bereitgestellt ist,
**dadurch gekennzeichnet, dass**
das Substrat (24) eine biegsame Vordruckschaltung, FPC, ist, die auf einer Seite, die sich von der Seite der Vielzahl von piezoelektrischen Wandlerelementen (23) unterscheidet, eine Vielzahl von Verdrahtungen in ringförmigen Formationen aufweist, und die eine Vielzahl von Elektroden, die den Wandlerelementen in der Vielzahl von piezoelektrischen Wandlerelementen (23) entsprechen, in der Formation von im Wesentlichen konzentrischen Kreisen parallel verbinden, wobei die Vielzahl von Verdrahtungen in ringförmigen Formationen die Vielzahl von Elektroden mit Antriebsschaltungen verbindet.

2. Ultraschallsonde (2) nach Anspruch 1, ferner zusätzlich zu einer ersten FPC (24), die als Substrat dient, eine zweite FPC (25) umfassend, die auf einer Rückfläche der ersten FPC (24) bereitgestellt ist, einen leitfähigen Teil in einer Position aufweist, die einer Position eines leitfähigen Teils der ersten FPC (24) entspricht, und dafür konfiguriert ist, ein Signal an die erste FPC (24) zu übertragen und von dieser zu empfangen, wobei
die leitfähigen Teile zwischen der ersten FPC (24) und der zweiten FPC (25) mittels einer leitfähigen Verbindung miteinander leitfähig gemacht sind, während andere Lücken als leitfähig verbundene Positionen mit einem nichtleitenden Klebemittel gefüllt sind.

3. Ultraschallsonde (2) nach einem der Ansprüche 1 bis 2, wobei jede von Gruppen von piezoelektrischen Wandlerelementen (23), die Elektroden entsprechen, die sich in im Wesentlichen konzentrischen Kreisen befinden, zu einer oder mehreren Antriebsschaltungen parallelgeschaltet ist.

4. Ultraschallsonde (2) nach Anspruch 3, wobei
unter den Gruppen von piezoelektrischen Wandlerelementen ein Verhältnis zwischen einer Anzahl von piezoelektrischen Wandlerelementen und einer Anzahl von Antriebschaltungen im Wesentlichen konstant gehalten wird.

5. Ultraschallsonde (2) nach Anspruch 3 oder 4, wobei für Gruppen von piezoelektrischen Wandlerelementen (23), die jeweils mit zwei oder mehr Antriebschaltungen verbunden sind, durchschnittliche Abstände von einem Mittelpunkt der Vielzahl von piezoelektrischen Wandlerelementen in im Wesentlichen konstanten Intervallen gehalten werden.

6. Ultraschallsonde (2) nach einem der Ansprüche 1 bis 5, ferner eine Schwingeinheit (28) umfassend, die dafür konfiguriert ist, die Vielzahl von piezoelektrischen Wandlerelementen (23), die in die Rasterabschnitte unterteilt sind, und das Substrat (24, 29) in Schwingungen zu versetzen.

7. Ultraschalldiagnosevorrichtung (1), umfassend:
eine Ultraschallsonde (2) nach einem der Ansprüche 1 bis 6; und
eine Erzeugungseinheit (552), die dafür konfiguriert ist, ein Ultraschallbild auf einer Grundlage eines durch die Ultraschallsonde (2) empfangenen Signals zu erzeugen.

8. Ultraschalldiagnosevorrichtung (1) nach Anspruch 7, ferner umfassend: einen Schwingmechanismus (7), der dafür konfiguriert ist, die Ultraschallsonde (2) in Schwingungen zu versetzen.

## Revendications

1. Sonde à ultrasons (2) comprenant :
une pluralité d'éléments transducteurs piézoélectriques (23) divisés en sections de grille, les éléments transducteurs piézoélectriques ayant une surface d'émission/réception d'ondes à ultrasons et une surface arrière opposée à la surface d'émission/réception d'ondes à ultrasons ;
les éléments transducteurs piézoélectriques (23) ayant des électrodes faisant face au côté de la surface d'émission/réception d'ondes à ultrasons et au côté de la surface arrière ; et
un substrat (24) fourni sur la surface arrière de la pluralité d'éléments transducteurs piézoélectriques (23),
**caractérisée en ce que**
le substrat (24) est un circuit pré-imprimé flexible, FPC, ayant une pluralité de fils en formations annulaires sur un côté différent du côté de la pluralité d'éléments transducteurs piézoélectriques (23) et qui connectent une pluralité d'électrodes correspondant aux éléments transducteurs dans la pluralité d'éléments transducteurs piézoélectriques (23) en parallèle dans la formation de cercles sensiblement concentriques, la pluralité de fils en formations annulaires connectant la pluralité d'électrodes à des circuits de pilotage.

2. Sonde à ultrasons (2) selon la revendication 1, comprenant en outre, en plus d'un premier FPC (24) servant de substrat, un deuxième FPC (25) qui est fourni sur une surface arrière du premier FPC (24), a une partie conductrice dans une position correspondant à une position d'une partie conductrice du premier FPC (24), et est configuré pour transmettre et recevoir un signal vers et depuis le premier FPC (24), dans laquelle
entre le premier FPC (24) et le deuxième FPC (25), les parties conductrices sont rendues conductrices l'une par rapport à l'autre par l'intermédiaire d'une jonction conductrice, tandis que des espaces autres que des positions jointes de manière conductrice sont remplis d'un agent adhésif non conducteur.

3. Sonde à ultrasons (2) selon l'une quelconque des revendications 1 à 2, dans laquelle chacun de groupes d'éléments transducteurs piézoélectriques (23) correspondant à des électrodes situées dans des cercles sensiblement concentriques est connecté en parallèle à un ou plusieurs circuits de pilotage.

4. Sonde à ultrasons (2) selon la revendication 3, dans laquelle,
parmi les groupes d'éléments transducteurs piézoélectriques, un rapport entre une quantité d'éléments transducteurs piézoélectriques et une quantité de circuits de pilotage est maintenu sensiblement constant.

5. Sonde à ultrasons (2) selon les revendications 3 ou 4, dans laquelle, pour des groupes d'éléments transducteurs piézoélectriques (23) connectés chacun à deux circuits de pilotage ou plus, des distances moyennes à partir d'un centre de la pluralité d'éléments transducteurs piézoélectriques sont maintenues à des intervalles sensiblement constants.

6. Sonde à ultrasons (2) selon l'une quelconque des revendications 1 à 5, comprenant en outre une unité de pivotement (28) configurée pour faire pivoter la pluralité d'éléments transducteurs piézoélectriques (23) divisés en les sections de grille et le substrat (24, 29).

7. Appareil de diagnostic par ultrasons (1) comprenant :
une sonde à ultrasons (2) selon l'une quelconque des revendications 1 à 6 ; et
une unité de génération (552) configurée pour générer une image à ultrasons sur la base d'un signal reçu par la sonde à ultrasons (2).

8. Appareil de diagnostic par ultrasons (1) selon la revendication 7, comprenant en outre : un mécanisme de pivotement (7) configuré pour faire pivoter la sonde à ultrasons (2).
